(19) Europäisches Patentamt
European Patent Office
Office européen des brevets

(11) Veröffentlichungsnummer: **0 136 472**
**B1**

# (12) EUROPÄISCHE PATENTSCHRIFT

(45) Veröffentlichungstag der Patentschrift: **08.02.89**

(51) Int. Cl.[4]: **C 12 N 15/00**, C 12 P 21/02, C 07 K 7/32, C 07 K 13/00, C 07 H 21/04 // C12R1/19

(21) Anmeldenummer: **84109276.0**

(22) Anmeldetag: **04.08.84**

(54) **Herstellung von Sekretin.**

(30) Priorität: **10.08.83 DE 3328793**

(43) Veröffentlichungstag der Anmeldung: **10.04.85 Patentblatt 85/15**

(45) Bekanntmachung des Hinweises auf die Patenterteilung: **08.02.89 Patentblatt 89/6**

(84) Benannte Vertragsstaaten: **AT BE CH DE FR GB IT LI LU NL SE**

(56) Entgegenhaltungen:
**EP-A- 0 095 351**

**PROCEEDINGS OF THE NATIONAL ACADEMY OF SCIENCES OF THE UNITED STATES OF AMERICA, Vol. 79, No. 8, April 1982, Baltimore (USA) M. SUZUKI et al. "Production in Escherichia coli of biologically active secretin, a gastrointestinal hormone" Seiten 2475-2479**
**NATURE, Vol. 298, No. 5874, 12. August 1982, New York/London A.F. BRADBURY et al. "Mechanism of the C-terminal amide formation by pituitary enzymes" Seiten 686-688**

(73) Patentinhaber: **HOECHST AKTIENGESELLSCHAFT, Postfach 80 03 20, D-6230 Frankfurt am Main 80 (DE)**

(72) Erfinder: **König, Wolfgang, Dr., Eppsteiner Strasse 25, D-6238 Hofheim am Taunus (DE)**
Erfinder: **Engels, Joachim, Dr., Feldbergstrasse 1, D-6242 Kronberg/Taunus (DE)**
Erfinder: **Uhlmann, Eugen, Dr., Fuchstanzstrasse 16, D-6240 Königstein/Taunus (DE)**
Erfinder: **Wetekam, Waldemar, Dr., Zeilring 40/I, D-6239 Eppstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

In der (nicht vorveröffentlichten) deutschen Patentanmeldung P 3 327 007.4 wurde ein Verfahren zur Herstellung eines Polypeptids der Formel I

$$Y-R-NH_2 \quad (I)$$

in der Y den Methioninrest oder einen über Methionin gebundenen Rest eines Bakterienproteins und R eine Peptidsequenz aus genetisch codierbaren Aminosäuren bedeutet, vorgeschlagen, das dadurch gekennzeichnet ist, dass man gentechnologisch ein Polypeptid der Formel II

$$Y-R-NH-CH_2-COOH \quad (II)$$

erzeugt und das Produkt enzymatisch in das Polypeptid der Formel I umwandelt. In einer bevorzugten Ausgestaltung dieses Verfahrens wird gentechnologisch ein Peptid der Formel II erzeugt, in dem R keinen Methioninrest enthält, und anschliessend wird der Rest Y durch Bromcyan-Spaltung abgetrennt.

Es wurde nun gefunden, dass nach diesem Verfahren auch Sekretin hergestellt werden kann.

Sekretin, ein Hormon aus dem Duodenum, ist ein Heptacosipeptid der Formel

H–His–Ser–Asp–Gly–Thr–Phe–Thr–Ser–Glu–Leu–Ser–Arg–Leu–Arg–Asp–Ser–Ala–Arg–Leu–Gln–Arg–Leu–Leu–Gln–Gly–Leu–Val–$NH_2$

(Eur. J. Biochem. 15, 1970, Seite 513–519).

Es stimuliert die Bicarbonatsekretion der Bauchspeicheldrüse und hemmt die gastrinstimulierte Magensäuresekretion. Aus diesen Gründen verspricht Sekretin ein gutes Arzneimittel bei gastrointestinalen Krankheiten, wie z.B. bei Läsionen im gastrointestinalen Trakt, zu werden.

Eine Therapie mit Sekretin scheitert jedoch an den hohen Gestehpreisen des isolierten natürlichen Sekretins, das nur in sehr geringer Menge in der Mukosa des Dünndarms vorkommt. Aus diesem Grunde wurden bereits zahlreiche Sekretinsynthesen beschrieben, die Sekretin in grösserer Menge bereitstellten, jedoch wegen des grossen Aufwands und der damit verbundenen hohen Kosten nicht befriedigen. So wurde Sekretin stufenweise unter Verwendung der p-Nitrophenylester-Methode (J. Am. Chem. Soc. 89, 1967, Seite 6753–6757), der «Repetitive Excess Mixed Anhydride» (REMA)-Methode (Helv. Chim. Acta 59, 1976, Seite 1112–1126; Int. J. Peptide Protein Res. 18, 1981, Seite 276–283) oder an fester Phase (Int. J. Peptide Protein Res. 9, 1977, Seite 63–70) synthetisiert. Die Verwendung von Segmenten zur Synthese von Sekretin bedarf möglichst racemisierungsfreier Kupplungsmethoden. So konnte bereits mit Hilfe der Azid-Methode (J. Am. Chem. Soc. 90, 1968, Seite 4711–4715) und der Dicyclohexylcarbodiimid/N-Hydroxysuccinimid-Methode (Chem. Ber. 105, 1972, Seite 2508–2514) Sekretin aufgebaut werden. Weitere Varianten der DCC-Kupplung bestanden in der Verwendung der racemisierungssenkenden und löslichkeitsvermittelnden Zusätze von 1-Hydroxybenzotriazol und 3-Hydroxy-4-oxo-3.4-dihydro-1.2.3-benzotriazin (Chem. Ber. 107, 1974, Seite 215–231; Gut Hormones, Ed. S.R. Bloom, 1978, Seite 165–168).

Demgegenüber stellt das erfindungsgemässe Verfahren eine leistungsfähige Methode dar, Sekretin in hoher Reinheit herzustellen.

Die gentechnologische Herstellung von Desamido-Sekretin in Escherichia coli ist bereits bekannt (M. Suzuki, S.-I. Sumi, A. Hasegawa, T. Nishizawa, K.-I. Miyoshi, S. Wakisaka, T. Miyake und F. Misoka, Proc. Natl. Acad. Sci. USA 79, 1982, Seite 2475–2479). Das synthetische Sekretingen wurde dort am N-terminalen Ende über ein Methionin mit dem β-Lactamasegen verbunden. Nach erfolgter Expression wurde die modifizierte β-Lactamase isoliert und das Peptid durch Bromcyanspaltung aus dem Protein isoliert.

Erfindungsgemäss wird demgegenüber Sekretylglycin gentechnologisch erzeugt, das enzymatisch in Sekretin umgewandelt wird. Vorteilhafte Ausgestaltungen dieser Erfindung werden im folgenden näher erläutert.

Der genetische Code ist bekanntlich «entartet», d.h. dass nur für zwei Aminosäuren eine einzige Nucleotid-Sequenz codiert, während den restlichen 18 genetisch codierbaren Aminosäuren 2 bis 6 Tripletts zuzuordnen sind. Für die Synthese des Gens besteht somit eine unübersehbare Vielfalt von Codon-Möglichkeiten. Die Erfindung betrifft nun eine besonders vorteilhafte DNA-Sequenz, die im Schema 1 (Anhang) niedergelegt und im folgenden auch als DNA-Sequenz I bezeichnet ist.

Am 5′-Ende des codierenden Stranges befindet sich eine «überhängende» DNA-Sequenz, entsprechend der Restriktionsendonuclease EcoRI, am 3′-Ende des codierenden Stranges dagegen die einzelsträngige, überhängende Sequenz entsprechend dem Restriktionsenzym Hind III. Diese beiden unterschiedlichen Erkennungssequenzen gewährleisten die Insertion der DNA in Plasmide in der gewünschten Orientierung.

Zwischen diesen Erkennungssequenzen und den Codons für die Aminosäuren-Folge befindet sich am 5′-Ende des codierenden Stranges das Codon für die Aminosäure Methionin. Am Ende dieses Stranges folgen auf das für Valin codierende Triplett das Codon für Glycin und vorteilhaft 2 Terminations-Tripletts.

Es wurde innerhalb des Strukturgens eine Reihe von singulären Erkennungssequenzen für Restriktionsendonucleasen eingebaut, die einerseits einen Zugang zu Teilsequenzen des Sekretin schaffen und andererseits die Durchführung von Mutationen erlauben. Diese Schnittstellen sind in die DNA-Sequenz des Schemas 1 eingetragen.

Die DNA-Sequenz I lässt sich aus 8 Oligonukleotiden mit unterschiedlicher Länge von Nukleotiden aufbauen, indem diese zunächst chemisch synthetisiert und dann über «sticky ends» von 6 Nukleotiden enzymatisch verknüpft werden.

Bei der DNA-Sequenz I wurde weiterhin berücksichtigt, dass bei denjenigen Aminosäuren, denen mehrere Codons zuzuordnen sind, diese nicht

gleichwertig sind, sondern vielmehr in der jeweiligen Wirtszelle wie E. coli unterschiedliche Präferenzen zeigen. Weiterhin wurden palindromische Sequenzen auf ein Mindestmass reduziert.

Die Genstruktur der DNA-Sequenz I ist somit leicht aus relativ kleinen Bausteinen zugänglich, ermöglicht die Subklonierung von Genfragmenten in gut bekannte Vektoren und erlaubt deren Expression in hoher Ausbeute.

Schema 2 zeigt die Nucleotidsequenz des Schemas 1, wobei jedoch die Genbausteine Ia bis Ih hervorgehoben sind, die zur Synthese des Gens dienen.

Diese Oligonucleotidbausteine können mit bekannten Synthesetechniken hergestellt werden (S.A. Narang, Tetrahedron 39, 1983, Seite 3; S.J. Beaucage und M.H. Caruthers, Tetrahedron Lett. 22, 1981, Seite 1859). Verwendet wurde die Phosphitmethode, wobei als Schutzgruppen an den Heterocyclen Benzoyl für Cytosin und Adenin, sowie Isobutyroyl für Guanin, Dimethoxytrityl an der 5'-OH-Gruppe der Desoxyribose und Methyl als Esterschutzgruppe am Phosphor eingesetzt wurden. Die Aktivierung der Phosphite fand mit Tetrazol (J.L. Fourrey und D.J. Shire, Tetrahedron Lett. 1981, Seite 729) statt. Die Reaktion wurde an Kieselgel als polymerem Träger durchgeführt und es wurden jeweils monomere Nucleosidphosphite addiert. Die Aufarbeitung erfolgte durch Dealkylierung der Methylestergruppen am Phosphor (G.W. Daub und E.E. van Tamelen, J. Amer. Chem. Soc. 99, 3526 (1977), Abspalten des Oligonucleotids vom Träger und Entfernen der Amidschutzgruppe mit konzentriertem wässrigem Ammoniak. Die Reinigung der Oligonucleotide erfolgte entweder mittels HPLC auf Reversed Phase- oder Ionenaustauschersäulen oder durch Acrylamidgelelektrophorese (Chemical and Enzymatic Synthesis of Gene Fragments, Ed. H.G. Gassen und A. Lang, Verlag Chemie, Weinheim 1982, Seite 177).

Die so gewonnenen Oligonucleotide werden dann am 5'-Ende mit Polynucleotidkinase phosphoryliert und in bekannter Weise mit T-4-DNA-Ligase enzymatisch verknüpft (V. Sgaramella und H.G. Khorana, J. Mol. Biol. 72, 427 (1972).

Dieses so erhaltene synthetische Gen ist ebenfalls Gegenstand der Erfindung.

Die Klonierung des resultierenden Gens für das Secretylglycin wurde in Plasmiden wie pBR 322 nach bekannten Methoden (Molecular Cloning, T. Maniatis, E.F. Fritsch und J. Sambrook, Cold Spring Harbor, 1982, Seite 211) durchgeführt. Neben der Möglichkeit der direkten Expression des Secretylglycins in E. coli wird das Strukturgen beispielsweise im pBR 322 an das Ende des β-Galactosidasegens als Fusionspeptid in ein β-Galactosidase produzierendes Plasmid ligiert. Hierfür bedarf es zweier weiterer Oligonucleotide als Linker, um das Strukturgen in das Plasmid zu insertieren.

Hierbei entsteht ein Gen, das die Nucleotidsequenz für die 1005 N-terminalen Aminosäuren der β-Galactosidase und die Nucleotidsequenz für Methionyl-sekretylglycin enthält. Als Wirtszelle für die Transformation des oben beschriebenen Plasmids dient E. coli. Exprimiert wird ein Protein, das N-terminal die Aminosäuresequenz der β-Galactosidase-(1-1005) und C-terminal die Sequenz für Methionyl-sekretyl-glycin enthält. Eine Bromcyanspaltung setzt aus diesem Protein Sekretyl-glycin frei.

Die das synthetische Gen enthaltenden Hybridplasmide, die damit transformierten Wirtsorganismen, das exprimierte Methionyl-sekretyl-glycin und die entsprechenden Fusionsproteine sowie das hieraus hergestellte Sekretylglycin sind ebenfalls Gegenstand der Erfindung.

Das Sekretylglycin ist in seinem chromatographischen Verhalten hydrophiler als Sekretin. Die biologische Aktivität des Sekretylglycins ist mit etwa 800 KE/mg deutlich schwächer als die des Sekretins. Als Test für die biologische Wirkung diente die Stimulierung der Pankreassekretion am Hund.

Mit Hilfe des amidbildenden Enzyms (Nature 298, 1982, Seite 686–688) konnte aus dem Sekretylglycin nach nochmaliger chromatographischer Reinigung voll aktives Sekretin mit etwa 4000 KE/mg Protein gewonnen werden.

In den folgenden Beispielen beziehen sich Prozentangaben auf das Gewicht (sofern nichts anderes angegeben ist).

Beispiel 1

Synthese der in Schema 2 beschriebenen Oligonucleotide

Die Synthese der vollständig geschützten Nucleotide wurde nach folgendem Schema durchgeführt:

Käufliches FRACTOSIL® (Merck, Darmstadt) wurde nach bekannten Methoden in das Propylamino-Silicagel überführt (Chemical and Enzymatic Synthesis of Gene Fragments, Ed. H.G. Gassen und A. Lang, Verlag Chemie 1982, Seite 71). Ein 5'-O-Dimethoxytrityl-nucleosid-3'-O-succinat wurde mit Dicyclohexylcarbodiimid an das Propylamino-Silicagel als Amid verknüpft (s. obige Lit.).

Die Darstellung der Oligonucleotide erfolgte nach folgendem Zyklus:

In einer Fritte werden 100 mg (2–4 µmol Nucleosid) des oben hergestellten Polymers nacheinander wie folgt behandelt:

1. zweimal waschen mit Nitromethan, das 1% Wasser enthält;

2. man lässt drei- bis fünfmal mit einer gesättigten Zinkbromidlösung in Nitromethan/1% Wasser jeweils zwei Minuten stehen und saugt anschliessend ab;

3. zweimal waschen mit Methanol;

4. zweimal waschen mit Tetrahydrofuran;

5. zweimal waschen mit Acetonitril;

6. mit 60–70 mg Nucleosidphosphit und 40 mg Tetrazol in 1 ml wasserfreiem Acetonitril fünf Minuten stehen lassen und absaugen;

7. man lässt mit einer Mischung aus 20%igem Acetanhydrid in Tetrahydrofuran/Lutidin/Dimethylaminopyridin (5:4:1/v,v,v) zwei Minuten stehen und saugt ab;

8. zweimal waschen mit Tetrahydrofuran;

9. mit einer Mischung aus Tetrahydrofuran/Wasser/Lutidin (2:1:2/v,v,v) zwei Minuten stehen lassen und absaugen;
10. mit einer Mischung aus 3% Jod in Tetrahydrofuran/Wasser/Collidin (1:4:5/v,v,v) zwei Minuten stehen lassen und absaugen;
11. zweimal waschen mit Tetrahydrofuran;
12. zweimal waschen mit Methanol;
13. zurück zu 1.

Der Synthesezyklus wird dann mit dem jeweiligen Nucleosidphosphit wiederholt, bis die Kette vollendet ist. Die Effizienz jedes Kupplungsschrittes wird spektroskopisch anhand der Absorption des Dimethoxytritylkations bei 494 nm vermessen, das bei der Abspaltung der Schutzgruppe mit der Zinkbromidlösung entsteht. Nach Vollendung der Synthese wird das völlig geschützte Oligonucleotid mit 2,4,6-Trimethylthiophenol/Triethylamin (1:1/v,v) eine Stunde behandelt, wobei die Phosphorsäuremethylester dealkyliert werden. Anschliessend wird mit gesättigter wässriger Ammoniaklösung innerhalb von drei Stunden das noch patiell geschützte Nucleotid vom Kieselgel abgespalten. Die ammoniakalische Lösung wird filtriert und noch 50–60 Stunden bei Raumtemperatur stehen gelassen, um das Oligonucleotid völlig von den restlichen Schutzgruppen zu befreien.

Die deblockierten Nucleotide werden entweder durch HPLC an Reversed Phase-Säulen (Laufmittel: 0,1 M Triethylammoniumacetat in Wasser gegen Acetonitril-Gradient; Chemical and Enzymatic Synthesis of Gene Fragments, Ed. H.G. Gassen und A. Lang, Verlag Chemie, Weinheim 1982, Seite 177), oder durch Gelelektrophorese an 20% Acrylamid, 7M Harnstoff-Gelen mit 0,1 M Trisboratpuffer, pH 8,3 (obige Lit., Seite 37) gereinigt.

Nach diesem Verfahren wurden die in Schema 2 niedergelegten Oligo-Nucleotide Ia bis Ih sowie die folgenden Nucleotide IIa und IIb hergestellt:

IIa: 5′ AGCTTGACGCG
IIb: 3′ ACTGCGCTTAA

Beispiel 2

Phosphorylierung der Oligonucleotidblöcke

Die Konstruktion des Methionyl-sekretylglycingens als Duplex ist in Schema 2 dargestellt.

3 µg Oligonucleotid werden mit 0,03 ml einer Lösung versetzt, die 10 mM an $MgCl_2$, 50 mM an Tris-Puffer, 0,2 mM an ATP und 20 mM an Dithiothreitol (DTT) ist und 5 Einheiten (Progress in Nucleid Acid Research 2, 1972, Seite 815) T4 Polynucleotidkinase enthält. Der Reaktionsablauf wurde durch Chromatographie auf Diethylaminoethyl-(DEAE)-Papier oder Polyethylenimin-(PEI)-Cellulose bestimmt. Die erhaltene Lösung wird direkt ohne Reinigung weiter verarbeitet.

Beispiel 3

Ligation der Nucleotid-Blöcke Ia–Ih

Jeweils 500–800 pmol 5′-phosphoryliertes Ib–Ig sowie 1,2 Äquivalente 5-OH-Ia und -Ih werden separat als Ia/b, Ic/d, Ie/f und Ig/h bei 95 °C zwei Minuten in Wasser erhitzt und langsam auf Raumtemperatur abgekühlt.

Die Proben werden dann alle vereint, lyophilisiert und in 0,05 ml 0,02 M HEPES (2-[4-(Hydroxyethyl)-1-piperazinyl]-ethansulfonsäure)-Puffer (pH 7,6) aufgenommen. Dazu gibt man 200 Einheiten (J. Biol. Chem. 243, 1968, Seite 4543) T4-DNA-Ligase. Nach 16 Stunden Inkubation bei 14 °C wird die Reaktion durch Erhitzen auf 80 °C beendet. Anschliessend wird auf einem 10% Polyacrylamidgel (ohne Harnstoff) das Produkt als Doppelstrang mit 102 Basenpaaren (Vergleich durch Hae III-Verdauung von pBR 322) isoliert und gereinigt.

Aufarbeitung: s. Molecular Cloning, Seite 173.

Beispiel 4

Klonierung des Methionyl-sekretylglycin-gens in pUC 8

pUC 8 (P.L. Biochemicals, GmbH,) wurde mit den Restriktionsendonucleasen Eco RI und Hind III gemäss Standardbedingungen (New England Biolaps, Inc., Beverly, MD, USA) gespalten und das grosse Fragment durch Agarose-Gelelektrophorese (1% niedrigschmelzende Agarose: Bethesda Research Laboratories, Inc., Gaithersberg, MD, USA) in 0,1 M Tris-borat (2,5 mM EDTA) gereinigt. Die grosse Bande wurde durch Schmelzen der Agarose bei 65 °C isoliert. Mit Phenol/Chloroform (1:1 v/v) wird extrahiert. Die wässrige Phase wird mit Ethanol versetzt. Der Niederschlag wird abzentrifugiert.

Das in Beispiel 3 synthetisierte Methionyl-secretylglycin-Gen (10 µg) wird mit 20 µg des Eco RI/Hind III-Fragmentes von pUC 8 in 0,02 ml eines 5 mM Tris-Puffers (pH 7,6, 10 mM Mg $Cl_2$, 1 mM ATP, 20 mM DTT und 200 Einheiten T4 DNA-Ligase) 12 Stunden bei 14 °C inkubiert.

Die Transformation in E. coli K 12 wurde nach bekannten Methoden durchgeführt (Proc. Natl. Acad. Sci. USA 69, 1972, Seite 2110–2114) und die resistenten Transformanden gegen 25 µg/ml Ampicillin isoliert. Eine weitere Analyse selektiver Transformanden wurde mittels der Behandlung mit Restriktionsenzymen durchgeführt. Es wurden drei Klone isoliert, die nach der Verdauung mit Eco RI und Hind III eine Insertion im Bereich von 102 Basenpaaren (Bp) enthielten. Weiter waren auch Schnittstellen für Kpn I und Pst I vorhanden.

Die Sequenzierung nach A.M. Maxam und W. Gilbert (Methods of Enzymology 65, 1980, Seite 499) bestätigte die synthetisierte Sequenz des Methionyl-secretylglycin-Gens.

Beispiel 5

Fusion des Methionyl-sekretylglycin-Gens mit dem β-Galactosidase-Gen von E. coli

Zur Expression wurde das beschriebene Methionyl-sekretylglycin-Gen in das Plasmid pWH 10 kloniert. Dieser Plasmidvektor wird nach gängigen Verfahren (K. Itakura, T. Hirose, R. Crea, A.D. Riggs, H.L. Heyneker, F. Bolivar, H.W. Boyer, Science 1977, Seite 1056) zum einen Teil aus dem E. coli-Plasmid pBR 322 und aus dem β-Galactosidase-Gen plus Regulationseinheit zum anderen Teil zusammengesetzt. 10 µg pBR 322 werden mit den Restriktionsendonucleasen Eco RI und Pvu II verdaut und auf 5% Polyacrylamidgel nach Standardbedingungen getrennt. Die mit Ethidiumbromid sichtbar gemachte Bande (2292 Bp) wurde aus dem Gel geschnitten und elektro-eluiert.

Aus dem Transduktionsphagen ∅ 80 dlac wurde mit der Endonuclease Eco RI und einer Partialverdauung durch Pvu II das Gen und seine Regulationseinheit für die β-Galactosidase herausgeschnitten (3185 Bp). Dieses Fragment trägt die Lactaseregulationsregion und das Gen für die β-Galactosidase von Aminosäure 1-1005. Dieses Lac-DNA-Fragment lässt sich mit dem DNA-Fragment von pBR 322 ligieren. Es resultiert ein Plasmid mit der Regulationseinheit des Lac-Operons, dem Strukturgen für die β-Galactosidase von 1–1005, dem Ampicillinresistenzgen von pBR 322 und dessen Replikationsregion. Dieses Lac-Operon kann durch beliebige andere Regulationsregionen wie z.B. tac ersetzt werden. Dieses Plasmid pWH 10 enthält bei Aminosäure 1004 der Galactosidase eine singuläre Eco RI Schnittstelle, die als Klonierungsstelle benutzt wird. Das Strukturgen des Methionyl-sekretylglycins aus 97 Basenpaaren wird am 3′-Ende mit einem Hind III-Eco RI-Adaptor

IIa 5′ AGCTTGACGCG
IIb 3′ ACTGCGCTTAA
Hind III Eco RI

versehen und für die Integration in das Plasmid pWH 10 verwendet. Es wird mit ECO RI geschnitten und dann das DNA-Stück mit dem Gen für das Methionyl-sekretylglycin wie oben beschrieben hineinligiert.

Die Nucleotidsequenz des Methionyl-sekretylglycin-Gens ist so konzipiert, dass der Leserahmen der β-Galactosidase kontinuierlich in das Gen des Methionyl-sekretylglycins übergeht. Das Genprodukt aus einer solchen Konstruktion ist eine Fusion von 1005 aminoterminalen Aminosäuren der β-Galactosidase (Proc. Natl. Acad. Sci. USA 74, 1977, Seite 1507) mit dem C-terminalen Gen des Methionylsekretylglycin.

Die Orientierung, in der das Methionyl-sekretylglycin-Gen in das Plasmid pWH 10 insertiert wurde, liess sich mittels Restriktionsenzymverdauung feststellen.

Beispiel 6

Als Wirtszelle für die Transformation des oben beschriebenen Plasmids pWH 10 dient der E. coli-Stamm K 12.

Die Transformation wird nach den üblichen Methoden der Molekularbiologie durchgeführt (Molecular Cloning, Seite 211).

Beispiel 7

Isolierung des β-Galactosidase-Sekretylglycins

Die Bakterien werden in einem 30 Liter-Fermenter unter Standardbedingungen in einem Vollmedium oder einem synthetischen Medium und Supplementation bis zur gewünschten optischen Dichte herangezogen und mit einem geeigneten Induktor, z.B. Isopropyl-β-D-thiogalactosid, für zwei Stunden induziert. Anschliessend werden die Zellen mit 0,1 mM Benzylsulfonylfluorid und 0,1% Kresol abgetötet. Nach Abzentrifugieren oder Filtrieren der Zellen werden diese in einem wässrig-sauren Medium bei pH 3,0 mit einer FRENCH-Presse oder DYNO-Mühle® (Willy Bachofen, Basel) aufgeschlossen und alle unlöslichen Bestandteile abzentrifugiert. Der Überstand wird verworfen. Der Rückstand wird in 7M Guanidinhydrochlorid aufgenommen und bei 15 000 g zetrifugiert. Der Überstand wird abdekantiert und mit der fünffachen Menge Wasser verdünnt. Man stellt auf 0 °C ein und zentrifugiert den resultierenden Niederschlag ab. Dieser wird in 7M Guanidinhydrochlorid-Lösung gelöst und über SEPHADEX G 200® in 7M Guanidinhydrochlorid chromatographiert. Die das β-Galactosidase-Sekretylglycin enthaltende Fraktion wird mit der fünffachen Menge Wasser verdünnt und der Rückstand abzentrifugiert.

Ausbeute 10 g.

Beispiel 8

Sekretylglycin

Sekretylglycin wurde analog Proc. Natl. Acad. Sci. USA 79, 1982, S. 2475–2497 aus dem oben gewonnenen β-Galactosidase-Sekretylglycin durch Bromcyanspaltung erhalten und anschliessend durch Chromatographie an SP-SEPHADEX C-25 mit Ammoniumacetatpuffern (pH 6,8) steigender Molarität (von 0,05 M–0,1 M) gereinigt. Das Ammoniumacetat wurde durch dreimalige Gefriertrocknung entfernt.

Ausb. 50 mg (Proteingehalt laut Aminosäureanalyse 81%) Aminosäureanalyse (Hydrolyse: 24 Stunden in 6N HCl bei 120 °C):

Asp (2,01), Thr (2,03), Ser (3,43), Glu (2,76), Gly (3,07), Ala (1,00), Val (0,89), Leu (5,57), Phe (1,12), His (0,89), Arg (3,89).

DC auf Kieselgelplatte (60 F 254) in n-Butanol/Wasser/Pyridin/Eisessig = 60:24:20:6 Vol.%; $R_f$ = 0,138 Biologische Wirkung: ca. 800 KE/mg Pankreassekretion am Hund.

Beispiel 9

Sekretin

Das in den neurosekretorischen Granula-Fraktionen aus Hypothalami vorkommende Enzym, das aus C-terminal mit Glycin verlängerten Peptiden die entsprechenden Peptidamide liefert, wurde analog FEBS Letters 152, 1983, Seite 277–279 gereinigt. Diese Enzymproben können mit Enzymen, die nach Prolin spalten, verunreinigt sein, was im Falle des Sekretins nicht stört, da Sekretin kein Prolin enthält.

3 mg Sekretylglycin werden in 10 ml 10 mM Phosphatpuffer (pH 7) gelöst und unter Rühren an der Luft und bei 37 °C mit einer gereinigten Enzymfraktion inkubiert. Nach 5 Stunden Reaktionszeit wird die Lösung gefriergetrocknet und analog Beispiel 8 chromatographisch gereinigt.

Ausb. 1,2 mg (Proteingehalt laut Aminosäureanalyse 82%) Aminosäureanalyse: Hydrolyse: 24 Stunden in 6N HCl bei 120 °C.

Asp (1,99), Thr (1,91), Ser (3,52), Glu (2,99), Gly (1,99), Ala (1,01), Val (1,03), Leu (5,88), Phe (0,97), His (0,89), Arg (3,91).

DC auf Kieselgelplatte (60 F 254) in n-Butanol/Wasser/Pyridin/Eisessig = 60:24:20:6 Vol.%; $R_f$ = 0,189. Der $R_f$-Wert ist mit synthetischem Sekretin (Gut Hormones, Hrsg. S.R. Bloom, 1978, Seite 165–168) identisch. Die biologische Wirkung entspricht etwa 4000 KE/mg Protein und ist mit synthetischem Sekretin vergleichbar.

Schema 1:

```
5' AATTC  ATG CAT  TCT GAC  GGT –
        G  TAC GTA  AGA CTG  CCA –
   Eco RI    Ava III

   ACC  TTC ACT TCT GAA CTG
   TGG  AAG TGA AGA CTT GAC
   Kpn I

   TCT  AGA  CTG CGT GAC TCT
   AGA ACA  GAC GCA CTG AGA
     Xba I

   GCT CGA CTC CAG CGT CTG
   CGA GCT GAG GTC GCA GAC
       Xho I

   CTG CAG  GGT CTG GTT GGT
   GAC GTC  CCA GAC CAA CCA
     Pst I

   TAG TA
   ATC ATT CGA
       Hind III
```

Schema 2:

```
     Ia
5'  AATTC ATG CAT TCT GAC GGT ACC –
3'        G TAC GTA AGA CTG CCA TGG –
     Ib

          Ic
   TTC ACT TCT GAA CTG TCT AGA
   AAG TGA AGA CTT GAC AGA TCT
                  Id

                  Ie
   CTG CGT GAC TCT  GCT CGA CTC
   GAC GCA CTG AGA CGA GCT GAC
                  If

                             Ig
   CAG CGT CTG CTG CAG GGT  CTC
   GTC GCA GAC GAC  GTC CCA GAC
                             Ih

   5' GTT GGT TAG TA
   3' CAA CCA ATC ATT CGA
```

**Patentansprüche für die Vertragsstaaten: BE, CH, DE, FR, GB, IT, LI, LU, NL, SE**

1. Verfahren zur Herstellung eines Polypeptids der Formel I

$$Y–R–NH_2 \qquad (I)$$

in der Y den Methioninrest oder einen über Methionin gebundenen Rest eines Bakterienproteins und R eine Peptidsequenz aus genetisch codierbaren Aminosäuren bedeutet, wobei man gentechnologisch ein Polypeptid der Formel II

$$Y–R–NH–CH_2–COOH \qquad (II)$$

erzeugt und das Produkt enzymatisch in das Polypeptid der Formel I umwandelt, dadurch gekennzeichnet, dass man gentechnologisch ein Polypeptid der Formel II erzeugt, in dem R die Peptidsequenz des Sekretin bedeutet, und das Produkt enzymatisch in das Polypeptid der Formel I umwandelt.

2. Verfahren nach Anspruch 1, dadurch gekennzeichnet, dass man aus dem Polypeptid der Formel I durch Bromcyan-Spaltung den Rest Y abtrennt.

3. Polypeptid der Formel II, in der Y den Methioninrest und R die Aminosäuresequenz des Sekretin bedeuten.

4. Polypeptid der Formel III,

$$Met–R–NH_2 \qquad (III)$$

in der R die Aminosäuresequenz des Sekretin bedeutet.

5. Polypeptid der Formel IV

$$H–R–Gly \qquad (IV)$$

in der R die Aminosäuresequenz des Sekretin bedeutet.

6. Fusionsproteine der Formel II,

$$Y–R–NH–CH_2–COOH \qquad (II)$$

in der Y einen über Methionin gebundenen Rest eines Bakterienproteins und R die Aminosäuresequenz des Sekretin bedeutet.

7. DNA-Sequenz der Formel V

```
5'–AA TTC ATG–        –GGT TAG TA
       G TAC–   (R)   –CCA ATC ATT CGA
```

in der R die Codons für die Aminosäuresequenz des Sekretin bedeuten.

8. Für das Verfahren nach Anspruch 1 geeignete Hybridplasmide, die zwischen einer EcoRI- und einer HindIII-Schnittstelle eine DNA-Sequenz der Formel V enthalten.

9. Wirtsorganismen, die Hybridplasmide nach Anspruch 8 enthalten.

**Patentansprüche für den Vertragsstaat Österreich:**

1. Verfahren zur Herstellung eines Polypeptids der Formel I

$$Y\text{–}R\text{–}NH_2 \qquad (I)$$

in der Y den Methioninrest oder einen über Methionin gebundenen Rest eines Bakterienproteins und R eine Peptidsequenz aus genetisch codierbaren Aminosäuren bedeutet, wobei man gentechnologisch ein Polypeptid der Formel II

$$Y\text{–}R\text{–}NH\text{–}CH_2\text{–}COOH \qquad (II)$$

erzeugt und das Produkt enzymatisch in das Polypeptid der Formel I umwandelt, dadurch gekennzeichnet, dass man gentechnologisch ein Polypeptid der Formel II erzeugt, in dem R die Peptidsequenz des Sekretin bedeutet, und das Produkt enzymatisch in das Polypeptid der Formel I umwandelt.

2. Verfahren nach Anspruch 1, dadurch gekennzeichnet, dass man aus dem Polypeptid der Formel I durch Bromcyan-Spaltung den Rest Y abtrennt.

**Claims for the contracting states: BE, CH, DE, FR, GB, IT, LI, LU, NL, SE**

1 A process for the preparation of a polypeptide of the formula I

$$Y\text{–}R\text{–}NH_2 \qquad (I)$$

in which Y is the methionine radical or a radical, bonded via methionine, of a bacterial protein and R denotes a peptide sequence of genetically codable amino acids, by producing a polypeptide of the formula II

$$Y\text{–}R\text{–}NH\text{–}CH_2\text{–}COOH \qquad (II)$$

by genetic engineering and converting the product enzymatically into the polypeptide of the formula I, characterized by producing a polypeptide of the formula II in which R denotes the peptide sequence of secretin by genetic engineering and converting the product enzymatically into the polypeptide of the formula I.

2. The process according to claim 1, characterized in that the radical Y is removed from the polypeptide of the formula I by cyanogen bromide cleavage.

3. The polypeptide of the formula II, in which Y denotes the methionine residue and R denotes the amino acid sequence of secretin.

4. The polypeptide of the formula III

$$Met\text{–}R\text{–}NH_2 \qquad (III)$$

in which R denotes the amino acid sequence of secretin.

5. The polypeptide of the formula IV

$$H\text{–}R\text{–}Gly \qquad (IV)$$

in which R denotes the amino acid sequence of secretin.

6. Fusion proteins of the formula II

$$Y\text{–}R\text{–}NH\text{–}CH_2\text{–}COOH \qquad (II)$$

in which Y denotes a residue, bonded via methionine, of a bacterial protein and R denotes the amino acid sequence of secretin.

7. The DNA sequence of the formula V

```
5'–AA TTC ATG–       –GGT TAG TA
                 (R)
        G TAC–       –CCA ATC ATT CGA
```

in which R denotes the codons for the amino acid sequence of secretin.

8. A hybrid plasmid which is suitable for the process according to claim 1, which contains a DNA sequence of the formula V between an EcoRI and a HindIII cutting site.

9. Host organisms containing a hybrid plasmid as claimed in claim 8.

**Claims for the contracting state: Austria**

1. A process for the preparation of a polypeptide of the formula I

$$Y\text{–}R\text{–}NH_2 \qquad (I)$$

in which Y is the methionine radical or a radical, bonded via methionine, of a bacterial protein and R denotes a peptide sequence of genetically codable amino acids, by producing a polypeptide of the formula II

$$Y\text{–}R\text{–}NH\text{–}CH_2\text{–}COOH \qquad (II)$$

by genetic engineering and converting the product enzymatically into the polapeptide of the formula I, characterized by producing a polypeptide of the formula II in which R denotes the peptide sequence of secretin by genetic engineering and converting the product enzymatically into the polypeptide of the formula I.

2. The process according to claim 1, characterized in that the radical Y is removed from the polypeptide of the formula I by cyanogen bromide cleavage.

**Revendications pour les Etats contractants: BE, CH, LI, DE, FR, GB, IT, LU, NL, SE**

1. Procédé de préparation d'un polypeptide répondant à la formule I

$$Y\text{–}R\text{–}NH_2 \qquad (I)$$

dans laquelle Y désigne le résidu méthionine ou un résidu lié par la méthionine d'une protéine bactérienne et R une séquence peptidique constituée d'aminoacides génétiquement codables, dans lequel on produit par génie génétique un polypeptide répondant à la formule II

$$Y\text{–}R\text{–}NH\text{–}CH_2\text{–}COOH \qquad (II)$$

et on transforme enzymatiquement le produit en le polypeptide répondant à la formule I, caractérisé en ce qu'on produit par génie génétique un polypeptide répondant à la formule II, dans laquelle R désigne la séquence peptidique de la sécrétine, et on transforme enzymatiquement le produit en le polypeptide répondant à la formule I.

2. Procédé suivant la revendication 1, caractérisé en ce qu'on sépare le résidu Y du polypeptide répondant à la formule I par coupure par le bromure de cyanogène.

3. Polypeptide répondant à la formule II, dans lequel Y désigne le résidu méthionine et R la séquence d'aminoacides de la sécrétine.

4. Polypeptide répondant à la formule III,

$$Met\text{–}R\text{–}NH_2 \qquad (III)$$

dans lequel R désigne la séquence d'aminoacides de la sécrétine.

5. Polypeptide répondant à la formule IV,

$$H\text{–}R\text{–}Gly \qquad (IV)$$

dans laquelle R désigne la séquence d'aminoacides de la sécrétine.

6. Protéines de fusion répondant à la formule IV,

$$Y\text{–}R\text{–}NH\text{–}CH_2\text{–}COOH \qquad (II)$$

dans laquelle Y désigne un reste lié par la méthionine d'une protéine bactérienne et R la séquence d'aminoacides de la sécrétine.

7. Séquence d'ADN répondant à la formule V,

```
5'–AA TTC ATG–   (R)   –GGT TAG TA
        G TAC–         –CCA ATC ATT CGA
```

dans laquelle R désigne les codons pour la séquence d'aminoacides de la sécrétine.

8. Plasmides hybrides appropriés pour le procédé suivant la revendication 1, qui contiennent, entre un site de coupure EcoRI et un site de coupre HindIII, une séquence d'ADN répondant à la formule V.

9. Organismes hôtes qui contiennent des plasmides hybrides selon la revendication 8.

**Revendications pour Etat contractant: Autriche**

1. Procédé de préparation d'un polypeptide répondant à la formule I

$$Y\text{–}R\text{–}NH_2 \qquad (I)$$

dans laquelle Y désigne le résidu méthionine ou un résidu lié par la méthionine et R une séquence peptidique d'aminoacides génétiquement codables, dans lequel on prépare par génie génétique un polypeptide répondant à la formule II

$$Y\text{–}R\text{–}NH\text{–}CH_2\text{–}COOH \qquad (II)$$

et on transforme enzymatiquement le produit en le polypeptide répondant à la formule I, caractérisé en ce qu'on produit par génie génétique un polypeptide répondant à la formule II, dans laquelle R désigne la séquence peptidique de la sécrétine, et on transforme enzymatiquement le produit en le polypeptide répondant à la formule I.

2. Procédé suivant la revendication 1, caractérisé en ce qu'on sépare le résidu Y du polypeptide répondant à la formule I par coupure par le bromure de cyanogène.